(19) Europäisches Patentamt
European Patent Office
Office européen des brevets

(11) EP 0 750 601 B1

(12) FASCICULE DE BREVET EUROPEEN

(45) Date de publication et mention de la délivrance du brevet:
**23.08.2000 Bulletin 2000/34**

(21) Numéro de dépôt: **96901385.3**

(22) Date de dépôt: **12.01.1996**

(51) Int Cl.[7]: **C07C 45/46**, C07C 49/84, B01J 29/90

(86) Numéro de dépôt international:
**PCT/FR96/00054**

(87) Numéro de publication internationale:
**WO 96/21637 (18.07.1996 Gazette 1996/33)**

# (54) PROCEDE FACILITANT LA REGENERATION D'UN CATALYSEUR A BASE D'UNE ZEOLITHE MIS EN OEUVRE DANS UNE REACTION D'ACYLATION ET UTILISATION

VERFAHREN ZUR ERLEICHTERUNG DER REGENIERUNG EINES ZEOLITHKATALYSATORS IN EINER ACYLIERUNGSREAKTION UND SEINE VERWENDUNG

PROCESS FACILITATING THE REGENERATION OF A ZEOLITHE-BASED CATALYST IMPLEMENTED IN AN ACYLATION REACTION AND UTILIZATION

(84) Etats contractants désignés:
**AT BE CH DE DK ES FR GB IE IT LI NL PT SE**

(30) Priorité: **13.01.1995 FR 9500346**

(43) Date de publication de la demande:
**02.01.1997 Bulletin 1997/01**

(73) Titulaire: **RHODIA CHIMIE**
**92408 Courbevoie Cédex (FR)**

(72) Inventeurs:
- **SPAGNOL, Michel**
  **F-69003 Lyon (FR)**
- **GILBERT, Laurent**
  **F-69007 Lyon (FR)**
- **LE GOVIC, Anne-Marie**
  **F-75010 Paris (FR)**

(74) Mandataire: **Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**EP-A- 0 334 096** **FR-A- 2 033 562**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet un procédé facilitant la régénération d'un catalyseur à base d'une zéolithe utilisé dans une réaction d'acylation.

Un autre objet de l'invention est un procédé d'acylation d'un éther aromatique, comportant dans une variante préférée, une étape additionnelle de régénération du catalyseur.

Plus particulièrement, l'invention a trait à un procédé d'acylation en para du groupe alkoxy porté par le noyau aromatique.

L'invention s'applique notamment à la préparation d'alkylcétones alkoxyaromatiques.

Les procédés classiques d'acylation des composés aromatiques, notamment des éthers de phénols, font appel à titre de réactif d'acylation, à un acide carboxylique, ou à l'un de ses dérivés tels que halogénure d'acide, ester ou anhydride.

La réaction est conduite généralement, en présence d'un catalyseur du type acide de Lewis (par exemple $AlCl_3$) ou du type acide de Brônsted ($H_2SO_4$, HF).

Depuis une dizaine d'années, on a proposé de mettre en oeuvre, les zéolithes, comme catalyseurs d'acylation.

Ainsi, on a décrit dans EP-A-0279322, la réaction en phase vapeur, d'un composé aromatique (vératrole) avec un dérivé d'acide carboxylique, en présence d'une zéolithe sous forme H telle que mordénite, faujasite et ZSM-5.

Il est également décrit dans US-A 4 960 943, un procédé d'acylation notamment de l'anisole, en présence de zéolithes ayant une taille de pores d'au moins 5 Angstrôms et qui répondent à la formule suivante : $M_{m/z}$ [$mMe^1O_2$. $nMe^2O_2$]. $qH_2O$] dans laquelle M est un cation échangeable, z est la valence du cation et $Me^1$ et $Me^2$ représentent les éléments du squelette anionique, n/m est un nombre entre 1-3000, de préférence, 1-2000 et q représente l'eau adsorbée.

Le développement à l'échelle industrielle des zéolithes se trouve limité par le fait que les zéolithes se désactivent relativement rapidement. En effet, la désactivation est attribuée, sans doute à l'empoisonnement du catalyseur par les produits organiques lourds formés au cours de la réaction, ce qui provoque une baisse du taux de conversion du substrat de départ entraînant ainsi une chute de la productivité.

Il se pose alors le problème de la réactivation du catalyseur qui implique souvent une procédure de régénération lourde, à haute température (température supérieure ou égale à 500°C) nécessitant un équipement spécifique.

Le but de l'invention est de fournir un procédé permettant d'obvier aux inconvénients précités.

La présente invention a précisément pour objet un procédé facilitant la régénération d'un catalyseur à base d'une zéolithe utilisé dans une réaction d'acylation caractérisé par le fait qu'il consiste à envoyer un flux gazeux chaud d'oxygène moléculaire ou d'un gaz en contenant sur un catalyseur qui est une zéolithe modifiée par ajout d'une quantité efficace d'au moins un élément métallique $M_1$ choisi parmi : le ruthénium, le rhodium, le palladium, l'osmium, l'iridium, le platine.

On a trouvé que le fait de doper la zéolithe par un élément de transition $M_1$ améliore la régénération du catalyseur qui peut être faite, préférentiellement, par une simple oxydation à l'air, à une température peu élevée.

On a constaté, de façon inattendue, que le fait de modifier la zéolithe par un élément de transition $M_1$ conduisait non seulement à une régénération aisée du catalyseur, mais aussi permettait d'obtenir au niveau de la réaction d'acylation d'un éther aromatique, un bon rendement réactionnel et une excellente sélectivité de la réaction.

Les zéolithes ainsi modifiées constituent d'excellents catalyseurs d'acylation d'un éther aromatique. L'invention réside donc également en un procédé d'acylation d'un éther aromatique suivie préférentiellement par une étape de régénération du catalyseur.

Selon une variante préférée du procédé de l'invention, on effectue d'abord l'acylation de l'éther aromatique en le faisant réagir avec un agent d'acylation, en présence d'une quantité efficace d'une zéolithe modifiée par au moins un élément de transition $M_1$ tel que défini, puis on procède après réaction, à la régénération du catalyseur, en le traitant thermiquement, par un gaz contenant de l'oxygène moléculaire.

Ce mode de mise en oeuvre préférentiel du procédé de l'invention permet non seulement d'avoir de bonnes performances au niveau de la réaction d'acylation mais il est également possible de régénérer très facilement le catalyseur.

Conformément au procédé de l'invention, on modifie la zéolithe utilisée comme catalyseur dans une réaction d'acylation, par un élément métallique choisi parmi le ruthénium, le rhodium, le palladium, l'osmium, l'iridium, le platine.

On peut également utiliser un mélange desdits éléments.

Comme éléments métalliques mis en oeuvre préférentiellement, on peut citer le palladium ou le platine.

La teneur de la zéolithe en élément métallique $M_1$ est généralement telle que le pourcentage pondéral du métal par rapport à la zéolithe soit compris entre 0,1 % et 25 %, de préférence entre 3 et 15%.

L'élément métallique $M_1$ est introduit dans la zéolithe par un échange cationique.

Par "zéolithe", on entend un tectosilicate cristallisé d'origine naturelle ou synthétique dont les cristaux résultent de l'assemblage tridimensionnel d'unités tétraédriques de $SiO_4$ et $TO_4$ : T représentant un élément trivalent tel que

aluminium, gallium, bore, fer, de préférence, l'aluminium.

Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau de canaux monodimensionnel, bidimensionnel ou tridimensionnel.

Dans le procédé de l'invention, on peut faire appel à une zéolithe naturelle ou synthétique.

Comme exemples de zéolithes naturelles susceptibles d'être utilisées, on peut citer, par exemple : la chabazite, la clinoptilolite, l'érionite, la mordénite, la phillipsite, l'offrétite.

Conviennent tout à fait bien à la mise en oeuvre de l'invention, les zéolithes synthétiques.

Comme exemples de zéolithes synthétiques à réseau monodimensionnel, on peut citer entre autres, la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel mises en oeuvre préférentiellement, on peut mentionner la mordénite, la ferrierite.

En ce qui concerne les zéolithes à réseau tridimensionnel, on peut nommer plus particulièrement, la zéolithe β, la zéolithe Y, la zéolithe X, la zéolithe ZSM-5, la zéolithe ZSM-11, l'offrétite.

On fait appel préférentiellement aux zéolithes synthétiques et plus particulièrement aux zéolithes qui sont sous les formes suivantes :

- la mazzite de rapport molaire Si/Al de 3,4,
- la zéolithe L de rapport molaire Si/Al de 1,5 à 3,5,
- la mordénite de rapport molaire Si/Al de 5 à 15,
- la ferrierite de rapport molaire Si/Al de 3 à 10,
- l'offrétite de rapport molaire Si/Al de 4 à 8,5.
- les zéolithes β de rapport molaire Si/Al supérieur à 8, de préférence, compris entre 10 et 35, et encore plus préférentiellement entre 12 et 35,
- les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60 ;
- la zéolithe X de type faujasite de rapport molaire Si/Al de 0,7 à 1,5,
- les zéolithes ZSM-5 ou silicalite d'aluminium de rapport molaire Si/Al de 10 à 500,
- la zéolithe ZSM-11 de rapport molaire Si/Al de 5 à 30.

Parmi toutes ces zéolithes, on fait appel préférentiellement dans le procédé de l'invention aux zéolithes β et Y.

Les zéolithes mises en oeuvre dans le procédé de l'invention, sont des produits connus décrits dans la littérature [cf. Atlas of zeolites structure types by W. M. Meier and D. H. Olson published by the Structure Commission of the International Zeolite Association (1978)].

On peut faire appel aux zéolithes disponibles dans le commerce ou bien les synthétiser selon les procédés décrits dans la littérature.

On peut se référer à l'Atlas précité, et plus particulièrement, pour la préparation :

- de la zéolithe L à la publication de Barrer R. M. et al, Z. Kristallogr., 128, pp. 352 (1969)
- de la zéolithe ZSM-12, au brevet US 3 832 449 et à l'article LaPierre et al, Zeolites 5, pp. 346 (1985),
- de la zéolithe ZSM-22, à la publication Kokotailo G.T. et al, Zeolites 5, pp. 349 (1985),
- de la zéolithe ZSM-23, au brevet US 4 076 842 et à l'article Rohrman A. C. et al, Zeolites 5, pp. 352 (1985),
- de la zéolithe ZSM-48, aux travaux de Schlenker J. L. et al, Zeolites 5, pp. 355 (1985),
- de la zéolithe β, au brevet US 3 308 069 et à l'article Caullet P. et al, Zeolites 12, pp. 240 (1992),
- de la mordénite, aux travaux de Itabashi et al, Zeolites 6, pp. 30 (1986),
- des zéolithes X et Y respectivement aux brevets US 2 882 244 et US 3 130 007,
- de la zéolithe ZSM-5, au brevet US 3 702 886 et à l'article Shiralkar V. P. et al, Zeolites 9, pp. 363 (1989),
- de la zéolithe ZSM-11, aux travaux de Harrison I. D. et al, Zeolites 7, pp. 21 (1987).

Les zéolithes peuvent être utilisées sous différentes formes dans le procédé de l'invention : poudre, produits mis en forme tels que granulés (par exemple, cyclindres ou billes), pastilles, monolithes (blocs en forme de nids d'abeilles) qui sont obtenus par extrusion, moulage, compactage ou tout autre type de procédé connu. En pratique, sur le plan industriel, ce sont les formes de granulés, de billes ou de monolithes qui présentent le plus d'avantages tant sur le plan de l'efficacité que sur le plan de commodité de mise en oeuvre.

L'invention n'exclut pas la présence de liants utilisés lors de la mise en forme de la zéolithe, par exemple des alumines ou des argiles.

Quelle que soit la zéolithe choisie, on fait un traitement si nécessaire qui la rend acide.

A cet effet, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $10^{-5}$ à $5.10^{-3}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

La zéolithe peut être également acidifiée en soumettant celle-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100ml/g. Ce passage peut être réalisé en une seule étape ou de préférence en plusieurs étapes successives.

Pour préparer les catalyseurs intervenant dans le procédé de l'invention, on peut recourir aux techniques classiques, connues en elles-mêmes, de préparation de catalyseurs métalliques supportés. On peut se référer, notamment, pour la préparation des différents catalyseurs à l'ouvrage : [J. F. LEPAGE "Catalyse de contact", conception, préparation et mise en oeuvre des catalyseurs industriels, Edition Technip (1978)].

Un mode de préparation des catalyseurs de l'invention consiste à déposer le ou les éléments $M_1$ sur la zéolithe, en précipitant les composés de manière en soi connue et en soumettant la masse de contact ainsi obtenue à un séchage et une calcination.

D'autres modes de préparation sont également possibles, en particulier un mode de fabrication desdits catalyseurs consiste à réaliser l'imprégnation de la zéolithe avec une solution d'imprégnation comprenant au moins un composé approprié des éléments métalliques choisis, dans un solvant volatil, de préférence l'eau, puis à sécher et calciner la masse de contact ainsi obtenue.

La zéolithe peut être chimiquement modifiée par imprégnation à sec ou voie humide.

Ainsi, un mode de préparation consiste à effectuer l'imprégnation à sec de la zéolithe, à l'aide d'une solution d'au moins un composé d'un élément transition $M_1$.

L'imprégnation est réalisée à sec c'est-à-dire que le volume total de solution utilisée est approximativement égal au volume poreux total présenté par la zéolithe. On sèche et calcine le produit obtenu.

Plus précisément, l'imprégnation à sec consiste à ajouter à une masse $m_1$ d'une poudre du produit à imprégner un volume V d'une solution aqueuse d'un ou des sels de cations ou d'anions à fixer sur la surface du solide. Le volume V de solution est choisie tel que $V/m_1$ soit égal au volume poreux à l'eau du solide à imprégner.

La concentration C en cations ou anions de la solution imprégnante est choisie telle que le rapport $CVM_2/m_1$ soit égal au pourcentage en poids choisi d'espèce imprégnante fixée sur la surface du produit à imprégner (avec $M_2$ = masse molaire de l'espèce imprégnante). On procède goutte à goutte à l'ajout de la solution de manière à obtenir une adsorption homogène.

Le produit peut ensuite être laissé au repos pendant un temps variable à température ambiante. Le produit est ensuite séché suivant les techniques classiques connues de l'Homme du Métier. Le séchage est généralement conduit à la pression atmosphérique ou sous pression réduite ou lyophilisation. Il peut être également calciné.

L'imprégnation voie humide se fait en dispersant la zéolithe, dans une solution aqueuse de sels de cations et/ou d'anions à fixer sur la surface du solide.

Cette solution peut présenter une concentration variant de $10^{-3}$ M à 10 M en l'espèce imprégnante.

Le pH de la solution pourra être avantageusement ajusté à une valeur au moins égale à celle du point isoélectrique du produit à modifier pour fixer préférentiellement les cations (cas usuel) ; toutefois, cette condition n'est pas impérative. Il est possible, au-dessous de ce point isoélectrique, de fixer correctement les cations lorsque les anions associés ont un caractère très "covalent".

La température de la solution peut varier de l'ambiante à 100°C.

La dispersion est agitée vigoureusement, pendant un temps variable.

Le produit est ensuite filtré et éventuellement lavé.

Selon les deux variantes telles que précédemment décrites, on obtient une zéolithe sur laquelle est déposée au moins un élément métallique $M_1$.

Le dépôt sur la zéolithe de plusieurs éléments métalliques peut bien entendu être réalisé successivement mais de de préférence, simultanément.

Dans les étapes suivantes, la zéolithe modifiée est soumise à une opération de séchage conduite avantageusement à une température variant de 50°C à 200°C, pendant une durée allant de préférence, de 2 à 12 heures.

En ce qui concerne l'opération de calcination, celle-ci est conduite à une température comprise entre 200°C et 700°C, de préférence entre 400°C et 600°C, pendant une durée variant de 1 à 15 heures, de préférence de 2 à 6 heures.

La nature des composés apportant les différents éléments utilisés pour la préparation des catalyseurs de l'invention n'est pas critique.

Les éléments métalliques $M_1$ peuvent être apportés sous forme d'un métal ou sous forme d'un dérivé inorganique tel qu'un oxyde ou un hydroxyde. Il est possible de faire appel à un sel minéral de préférence, nitrate, sulfate, oxysulfate, halogénure, oxyhalogénure, silicate, carbonate, ou à un dérivé organique de préférence, cyanure, oxalate, acétylacétonate ; alcoolate et encore plus préférentiellement méthylate ou éthylate ; carboxylate et encore plus préférentiellement acétate. Peuvent être également mis en oeuvre des complexes, notamment chlorés ou cyanés de métaux $M_1$ et/ou de métaux alcalins, de préférence sodium, potassium ou d'ammonium

A titre d'exemples de composés susceptibles d'être mis en oeuvre pour la préparation des catalyseurs de l'invention, on peut citer notamment :

- pour le ruthénium :
  - le chlorure de ruthénium (III)
  - le chlorure de ruthénium (IV)
  - le pentafluorure de ruthénium
  - l'hydroxyde de ruthénium (III)
  - l'oxyde de ruthénium (II)
  - l'oxyde de ruthénium (IV)
  - l'oxychlorure de ruthénium ammoniaqué $Ru_2(OH)_2Cl_4$, $7NH_3$, $5H_2O$
  - l'acétate de ruthénium
- pour le palladium :
  - le bromure de palladium (II)
  - le chlorure de palladium (II)
  - l'iodure de palladium (II)
  - le cyanure de palladium (II)
  - le nitrate de palladium (II) hydraté
  - le sulfate de palladium (II) dihydraté
  - le monoxyde de palladium (II) hydraté ou non
  - le dioxyde de palladium (IV) hydraté ou non
  - l'acétate de palladium (II)
  - l'acétylacétonate de palladium (II)
  - le tétrachloropalladate(II) d'ammonium
  - l'hexachloropalladate (IV) de potassium
  - le nitrate de tétramine de palladium (II)
  - le dichlorobis(acétonitrile) de palladium (II)
  - le dichlorobis(benzonitrile) de palladium (II)
  - le dichloro(1,5-cyclooctadiène) palladium (II)
  - le dichlorodiamine palladium (II)
- pour le platine :
  - le bromure de platine (II)
  - le chlorure de platine (II)
  - le chlorure de platine (IV)
  - l'iodure de platine (II)
  - l'hydroxyde de platine (II)
  - l'hydroxyde de platine (II) hydraté
  - le monoxyde de platine (II) hydraté ou non
  - le dioxyde de platine (IV) hydraté ou non
  - le cyanure de platine (II)
  - l'acétylacétonate de platine (II)

- le bis(oxalato)platinate (II) de potassium
- le tétrachloroplatinate (II) d'ammonium
- l'hexachloroplatinate (IV) d'ammonium
- le tétrachloroplatinate (IV) de sodium hydraté
- l'hexachloroplatinate (IV) de sodium hexahydraté
- l'hexachloroplatinate (IV) de potassium
- l'hexacyanoplatinate (IV) de potassium
- le chlorure de tétramine de platine (II) hydraté
- l'hydroxyde de tétramine de platine (II) hydraté
- le dichlorodiamine de platine (II)
- le tétrachlorodiamine de platine (IV)
- le dibromo(1,5-cyclooctadiène) platine (II)
- le dichloro(1,5-cyclooctadiène) platine (II)
- le dichlorobis(benzonitrile) de platine (II)
- le dichlorobis(pyridine) platine (II)
- l'acide chloroplatinique hexahydraté
- le chlorure de bis(éthylènediamine) de platine (II)

- pour le rhodium :

  - le bromure de rhodium (III)
  - le chlorure de rhodium (III) anhydre
  - le chlorure de rhodium (III) hydraté
  - l'iodure de rhodium (III)
  - le nitrate de rhodium (III)
  - le dioxyde de rhodium (IV) hydraté ou non
  - le sesquioxyde de rhodium (III) hydraté ou non
  - l'acétate de rhodium (II) dimère
  - l'acétylacétonate de rhodium (III)
  - l'hexachlororhodate (III) de sodium hydraté
  - l'hexachlororhodate (III) d'ammonium
  - l'acétylacétonatobis(éthylène) de rhodium (I)
  - l'hexanitritorhodate (III) de potassium

- pour l'osmium :

  - le chlorure d'osmium (II)
  - le chlorure d'osmium (III)
  - le chlorure d'osmium (IV)
  - l'iodure d'osmium (IV)
  - l'oxyde d'osmium (II)
  - l'oxyde d'osmium (III)
  - l'oxyde d'osmium (IV)

- pour l'iridium :

  - le bromure d'iridium (III)
  - le bromure d'iridium (IV)
  - le chlorure d'iridium (II)
  - le chlorure d'iridium (III)
  - le chlorure d'iridium (IV)
  - l'iodure d'iridium (III)
  - l'iodure d'iridium (IV)
  - le nitrate d'iridium (II) hydraté
  - le sulfate d'iridium (III) hydraté
  - le dioxyde d'iridium (IV) hydraté ou non
  - le sesquioxyde d'iridium (III) hydraté ou non
  - l'acétate d'iridium (II)

- le nitrate d'hexamine d'iridium (III)
- le chlorure d'hexamine d'iridium (III)

Le catalyseur obtenu consistant donc en une zéolithe modifiée et mis en oeuvre comme catalyseur d'acylation, est très facilement régénérée, en fin de réaction.

Par "réaction d'acylation", on entend une réaction bien connue de l'Homme du Métier qui consiste à faire réagir un substrat, de préférence, aromatique avec un agent d'acylation : la réaction pouvant être conduite en phase vapeur ou liquide.

Après élimination si nécessaire de la phase liquide, on envoie sur le catalyseur un flux gazeux chaud d'oxygène moléculaire ou d'un gaz en contenant.

Ce gaz peut être de l'oxygène pur ou de l'oxygène dilué avec un gaz inerte, par exemple l'azote ou un gaz rare, de préférence, l'argon. On fait appel préférentiellement à l'air.

Le débit du gaz est, par exemple, de 0,1 à 50 litres/heure.

La température du flux gazeux est avantageusement inférieure à 350°C, et est comprise de préférence entre 100°C et 250°C.

La durée du traitement peut varier largement, par exemple, entre 1 et 20 heures.

En fin de réaction, on récupère un catalyseur qui a repris toute son activité catalytique initiale.

On note donc que la régénération est effectuée à basse température contrairement à ce qui est fait habituellement avec une simple zéolithe.

Une illustration de la régénération d'un catalyseur zéolithique modifié est donné dans la réaction d'acylation d'un éther aromatique qui suit.

Par ailleurs, on a trouvé que la zéolithe dopée par un élément de transition $M_1$ était un très bon catalyseur de la réaction d'acylation d'un éther aromatique.

Un autre objet de la présente invention est un procédé d'acylation d'un éther aromatique qui consiste à faire réagir ledit éther aromatique avec un agent d'acylation, en présence d'un catalyseur, puis à récupérer le produit formé, ledit procédé étant caractérisé par le fait que l'on conduit la réaction d'acylation en présence d'une quantité efficace d'une zéolithe modifiée par au moins un élément de transition $M_1$.

Selon une variante préférée du procédé de l'invention, on effectue d'abord l'acylation de l'éther aromatique en le faisant réagir avec un agent d'acylation, en présence d'une zéolithe modifiée par un élément métallique $M_1$ tel que défini, puis on procède après réaction, à la régénération du catalyseur, en traitant thermiquement, ladite zéolithe par un gaz contenant de l'oxygène moléculaire.

Dans l'exposé qui suit de la présente invention, on entend "par éther aromatique", un composé aromatique dont un atome d'hydrogène directement lié au noyau aromatique est remplacé par un groupe éther et par "composé aromatique", la notion classique d'aromaticité telle que définie dans la littérature, notamment par Jerry MARCH, Advanced Organic Chemistry, 4ème édition, John Wiley and Sons, 1992, pp. 40 et suivantes.

Plus précisément, la présente invention a pour objet un procédé d'acylation d'un éther aromatique de formule générale (I):

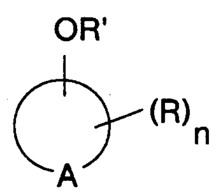

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- n est un nombre inférieur ou égal à 4.

Dans le présent texte, on désigne, de manière simplifiée, par "groupes alkoxy", les groupes du type R'-O- dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant cyclique.

L'éther aromatique qui intervient dans le procédé de l'invention répond à la formule (I) dans laquelle R' représente un radical aliphatique acyclique, saturé ou insaturé, linéaire ou ramifié.

Plus préférentiellement, R' représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène, un groupe carboxylique).

Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Le radical aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux éthers aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle, éthyle, propyle, isopropyle, butyle, isobutyle, sec-butyle, tert-butyle ou phényle.

Dans la formule générale (I) des éthers aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés.

Le reste A peut porter un ou plusieurs substituants sur le noyau aromatique.

Des exemples de substituants R sont donnés ci-après mais cette liste ne présente pas de caractère limitatif. N'importe quel substituant peut être présent sur le cycle dans la mesure où il n'interfère pas au niveau du produit désiré.

Le reste A pouvant entre autres porter plusieurs groupes alkoxy, il est possible selon le procédé de l'invention d'acyler des composés polyalkoxylés.

Le procédé de l'invention s'applique plus particulièrement, aux éthers aromatiques de formule (Ia):

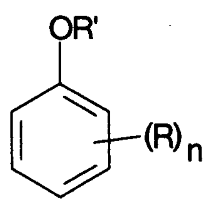

(Ia)

dans laquelle :

- n est un nombre inférieur ou égal à 4, de préférence égal à 0,1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,
- le ou les radicaux R représentent l'un des atomes ou groupes suivants :

  - . un atome d'hydrogène,
  - . un radical alkyle, linéaire ou ramifié, ayant de 1 à 6 atomes de carbone, de préférence de 1 à 4 atomes de

carbone, tel que méthyle, éthyle, propyle, isopropyle, butyle, isobutyle, sec-butyle, tert-butyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un atome d'halogène, de préférence un atome de fluor, chlore ou brome, un radical trifluorométhyle.

- les radicaux R' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux R' et R peuvent représenter un radical méthylène dioxy ou éthylène dioxy.

Le procédé de l'invention s'applique plus particulièrement aux éthers aromatiques de formule (Ia) dans laquelle n est égal à 1, les radicaux R et R' représentant tous deux, des radicaux alkoxy, identiques ou différents.

A titre illustratif de composés répondant à la formule (I), on peut mentionner plus particulièrement :

- des monoéthers tels que l'anisole, l'éthoxybenzène (phénétole), le butoxybenzène, l'isobutoxybenzène, le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-benzylanisole, le 2-cyclohexylanisole, le 1-bromo 2-éthoxybenzène, le 1-bromo 3-éthoxybenzène, le 1-chloro 2-éthoxybenzène, le 1-chloro 3-éthoxybenzène, le 1-éthoxy 2-éthylbenzène, le 1-éthoxy 3-éthylbenzène, le 2,3-diméthylanisole, le 2,5-diméthylanisole,
- des diéthers comme le vératrole, le 1,3-diméthoxybenzène, le 1,2-diéthoxybenzène, le 1,3-diéthoxybenzène, le 1,2-dipropoxybenzène, le 1,3-dipropoxybenzène, le 1,2-méthylènedioxybenzène, le 1,2-éthylènedioxybenzène,
- des triéthers comme le 1,2,3-triméthoxybenzène, le 1,3,5-triméthoxybenzène, le 1,3,5-triéthoxybenzène.

Les composés auxquels s'applique de manière plus particulièrement intéressante le procédé selon l'invention sont l'anisole et le vératrole.

Pour ce qui est du réactif d'acylation, il répond plus particulièrement à la formule (II):

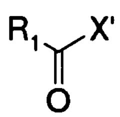

(II)

dans laquelle :

- $R_1$ représente :

  . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 3 à 8 atomes de carbone ; un radical aliphatique saturé ou insaturé, linéaire ou ramifié, porteur d'un substituant cyclique,

- X' représente :

  . un atome d'halogène, de préférence un atome de chlore ou de brome,
  . un groupe hydroxyle,
  . un radical -O-CO-$R_2$ avec $R_2$, identique ou différent de $R_1$, ayant la même signification que $R_1$ : $R_1$ et $R_2$ pouvant former ensemble un radical divalent aliphatique saturé ou insaturé, linéaire ou ramifié, ayant au moins 2 atomes de carbone.

Par substituant cyclique, on se réfère à ce qui est décrit précédemment.

Plus préférentiellement, $R_1$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène ou un groupe $CF_3$).

$R_1$ représente de préférence un radical alkyle ayant de 1 à 4 atomes de carbone, tel que méthyle, éthyle, propyle, isopropyle, butyle, isobutyle, sec-butyle, tert-butyle.

Le radical $R_1$ représente également d'une manière préférentielle un radical phényle qui peut être éventuellement substitué. N'importe quel substituant peut être présent sur le cycle dans la mesure où il n'interfère pas au niveau du produit souhaité.

Comme exemples plus particuliers de substituants, on peut citer, notamment :

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un groupe hydroxyle,
. un atome d'halogène, de préférence un atome de fluor, chlore ou brome.

Les agents d'acylation préférés sont les anhydrides d'acides. Ils répondent plus particulièrement à la formule (II) dans laquelle $R_1$ et $R_2$ sont identiques et représentent un radical alkyle ayant de 1 à 4 atomes de carbone.

Lorsque l'agent d'acylation est un halogénure d'acide, il répond préférentiellement à la formule (II) dans laquelle X' représente un atome de chlore et $R_1$ représente un radical méthyle ou éthyle.

A titre illustratif d'agents d'acylation répondant à la formule (II), on peut citer plus particulièrement :

- l'anhydride acétique,
- l'anhydride propanoïque,
- l'anhydride isobutyrique,
- l'anhydride trifluoroacétique,
- l'anhydride benzoïque.
- le chlorure d'acétyle,
- le chlorure de monochloroacétyle,
- le chlorure de dichloroacétyle,
- le chlorure de propanoyle,
- le chlorure d'isobutanoyle,
- le chlorure de pivaloyle,
- le chlorure de stéaroyle,
- le chlorure de crotonyle,
- le chlorure de benzoyle,
- les chlorures de chlorobenzoyle,
- le chlorure de p-nitrobenzoyle
- les chlorures de méthoxybenzoyle,
- les chlorures de naphtoyle,
- l'acide acétique.

Conformément à l'invention, la réaction d'acylation est conduite avantageusement en phase liquide comprenant l'éther aromatique et l'agent d'acylation, en présence du catalyseur.

L'un des réactifs de départ peut servir de solvant réactionnel mais il est également possible de faire appel à un solvant organique.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques.

A titre d'exemples d'hydrocarbures aliphatiques, on peut citer plus particulièrement les paraffines tels que notamment, l'hexane, l'heptane, l'octane, le nonane, le décane, le undécane, le dodécane, le tétradécane ou le cyclohexane, et le naphtalène et les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachloroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs

dibromobenzènes ; le 1-bromonaphtalène.

On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy 3-oxapentane) ; l'oxyde de benzyle ; le dioxane, le tétrahydrofuranne (THF).

On peut faire appel à des solvants aprotiques polaires tels que les composés nitrés comme par exemple, le nitrométhane, le nitroéthane, le 1-nitropropane, le 2-nitropropane ou leurs mélanges, le nitrobenzène ; les nitriles aliphatiques ou aromatiques comme l'acétonitrile, le propionitrile, le butanenitrile, l'isobutanenitrile, le benzonitrile, le cyanure de benzyle ; la tétraméthylène sulfone (sulfolane).

Les solvants préférés sont : le dichlorométhane, le tétrachlorométhane, le THF et l'oxyde de diéthyle.

On peut également utiliser un mélange de solvants organiques.

On utilise préférentiellement, le substrat de départ comme solvant réactionnel.

Dans une première étape du procédé de l'invention, on conduit l'acylation de l'éther aromatique. Selon une variante préférée, on effectue dans une étape suivante, la régénération du catalyseur.

Le rapport entre le nombre de moles d'éther aromatique et le nombre de moles d'agent d'acylation peut varier car le substrat peut servir de solvant réactionnel. Ainsi, le rapport peut aller de 0,1 à 10, et se situe de préférence entre 0,5 et 4,0.

La quantité de catalyseur que l'on met en oeuvre dans le procédé de l'invention peut varier dans de larges limites.

Lorsque l'on réalise le procédé en discontinu, le catalyseur peut représenter en poids par rapport à l'éther aromatique engagé, de 0,01 à 50 %, de préférence, de 1,0 à 20 %.

Cependant si l'on réalise le procédé en continu, par exemple en faisant réagir un mélange de l'éther aromatique et de l'agent d'acylation sur un lit fixe de catalyseur, ces rapports catalyseur/éther aromatique n'ont pas de sens et à un instant donné, on pourra avoir un excès pondéral de catalyseur par rapport à l'éther aromatique de départ. Dans ce cas, le temps de séjour du flux de matière sur le lit catalytique varie, par exemple, entre 5 mn et 10 heures, et de préférence, entre 15 mn et 3 heures, selon la température de la réaction et du taux de conversion souhaité.

Pour ce qui est de la quantité de solvant organique mis en oeuvre, elle est choisie généralement de telle sorte que le rapport entre le nombre de moles de solvant organique et le nombre de moles d'éther aromatique varie, de préférence, entre 0 et 100 et encore plus préférentiellement entre 0 et 50.

La température à laquelle est mise en oeuvre la réaction d'acylation dépend de la réactivité du substrat de départ et de celle de l'agent d'acylation.

Elle se situe entre 20°C et 300°C, de préférence entre 40°C et 200°C.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

D'un point de vue pratique, le procédé peut être mis en oeuvre en discontinu ou en continu.

Selon la première variante, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

L'autre variante de l'invention consiste à conduire la réaction en continu, dans un réacteur tubulaire comportant le catalyseur solide disposé en lit fixe.

L'éther aromatique et l'agent d'acylation peuvent être introduits séparément ou en mélange dans le réacteur.

Ils peuvent également être introduits dans un solvant tel que mentionné précédemment.

En fin de réaction, on récupère une phase liquide comprenant l'éther aromatique acylé qui peut être récupéré de manière classique, par distillation ou par recristallisation dans un solvant approprié, après élimination préalable des réactifs en excès.

Selon un mode préféré de réalisation de l'invention, on effectue dans une étape suivante du procédé de l'invention, une régénération du catalyseur utilisé.

Après élimination de la phase liquide, on envoie sur le catalyseur un flux gazeux chaud d'oxygène moléculaire ou d'un gaz en contenant.

La régénération du catalyseur est conduite comme décrite précédemment.

En fin de traitement, on récupère un catalyseur qui a repris toute son activité catalytique initiale.

Le procédé de l'invention est particulièrement bien adapté à la préparation de la 4-méthoxyacétophénone et de la 3,4-diméthoxyacétophénone dénommée couramment acétovératrole, par acétylation respectivement de l'anisole ou du vératrole.

Un avantage du procédé de l'invention est que la réaction d'acylation s'effectue sans qu'il y ait une O-désalkylation de l'éther aromatique de départ.

De plus, on note la présence de peu d'isomère ortho en raison du caractère para-orienteur du catalyseur.
Les exemples qui suivent, illustrent l'invention sans toutefois la limiter.
Dans les exemples, les rendements mentionnés correspondent à la définition suivante :

$$\text{Rendement : } RR_{A.A.} = \frac{\text{nombre de moles d'agent d'acylation introduites}}{\text{nombre de moles de composé aromatique acylé formées}}\ \%$$

Exemple 1

Préparation d'une zéolithe β modifiée par du palladium,

Dans un flacon de polyéthylène de 250 $cm^3$, on place :

- 39,5 $cm^3$ d'une solution d'hydroxyde de tétramine de palladium $Pd(NH_3)_4(OH)_2$ ; solution CLAL 8949 à 15,2 g/l de palladium,
- 60,5 $cm^3$ d'eau désionisée.

On ajoute ensuite, 20 g d'une poudre de zéolithe β sous forme $H^+$ commercialisée par VALDOR sous la dénomination (PQ)CBV 811-25.
On laisse 24 heures sous agitation, à température ambiante (20°C).
On sépare la zéolithe modifiée par centrifugation, on la sèche à l'étuve à 110°C puis on la calcine à 450°C, pendant 2 heures.
On obtient une zéolithe comprenant 3 % en poids de palladium.

Exemple 2

Dans un réacteur tubulaire, chauffé par une double enveloppe, on introduit 13 ml de zéolithe Pd (3%)/Hβ (soit environ 5 g) sous forme de poudre préparée selon l'exemple 1.
La double enveloppe est chauffée à 100°C et l'on introduit alors par le bas du réacteur, à l'aide d'une pompe HPLC, un mélange d'anisole et d'anhydride acétique mis en oeuvre dans un rapport molaire de 2, avec un débit de 0,2 ml/mn.
Le mélange réactionnel est soutiré en continu par débordement.
On suit le rendement de la réaction au cours du temps par prélèvement d'aliquotes qui sont ensuite analysés par chromatographie en phase vapeur.
Après 100 heures de réaction, le rendement en acétoanisole a chuté à environ 10%.

Tableau (I) :

| activité initiale | |
|---|---|
| Temps (h) | Rendement (% / anhydride acétique) |
| 3,5 | 72 |
| 7,5 | 73 |
| 30 | 50 |
| 51,2 | 26,2 |
| 70 | 20 |
| 94,3 | 13 |
| 100 | 10 |

On vidange le réacteur et l'on réactive le catalyseur sous air à 190°C pendant 3 heures.
On reprend alors l'injection du mélange anisole/anhydride acétique (2/1) à 100°C.
On obtient les performances suivantes:

Tableau (II) :

| après réactivation (sous air, 3 h à 190°C) | |
|---|---|
| Temps (h) | Rendement (% / anhydride acétique) |
| 2,6 | 73,2 |
| 11 | 58 |
| 36 | 34,5 |
| 51 | 24 |
| 70 | 20 |
| 93 | 13 |

Exemple 3

Dans un réacteur tubulaire, chauffé par une double enveloppe, on introduit 13 ml de zéolithe Pd (3%)/Hβ (soit environ 5 g) préparée selon l'exemple 1, sous forme d'extrudés contenant 40% de liant alumine.

La double enveloppe est chauffée à 105°C et l'on introduit alors par le bas du réacteur, à l'aide d'une pompe HPLC, un mélange d'anisole et d'anhydride acétique mis en oeuvre dans un rapport molaire de 2, avec un débit de 0,2 ml/mn.

Le mélange réactionnel est soutiré en continu par débordement.

On suit le rendement de la réaction au cours du temps par prélèvement d'aliquotes qui sont ensuite analysés par chromatographie en phase vapeur.

Après 120 heures de réaction, le rendement en acétoanisole a chuté à environ 13%.

Tableau (III):

| activité initiale | |
|---|---|
| Temps (h) | Rendement (% / anhydride acétique) |
| 4 | 70 |
| 5 | 75 |
| 30 | 40 |
| 53 | 29 |
| 70 | 24,7 |
| 100 | 23,2 |
| 120 | 13,4 |

On vidange le réacteur et l'on réactive le catalyseur sous air à 180°C pendant 3 heures.

On reprend alors l'injection du mélange anisole/anhydride acétique (2/1) à 105°C.

On obtient les performances suivantes:

Tableau (IV) :

| après réactivation (sous air, 3 h à 180°C) | |
|---|---|
| Temps (h) | Rendement (% / anhydride acétique) |
| 4,3 | 53 |
| 6 | 56 |
| 21,3 | 32,5 |
| 23,3 | 31,4 |

**Revendications**

1. Procédé de régénération d'un catalyseur à base d'une zéolithe utilisé dans une réaction d'acylation caractérisé par le fait qu'il consiste à envoyer un flux gazeux chaud d'oxygène moléculaire ou d'un gaz en contenant sur un catalyseur qui est une zéolithe modifiée par ajout d'une quantité efficace d'au moins un élément métallique $M_1$ choisi parmi : le ruthénium, le rhodium, le palladium, l'osmium, l'iridium, le platine.

2. Procédé selon la revendication 1 caractérisé par le fait que le catalyseur est une zéolithe modifiée par l'élément métallique $M_1$ choisi parmi le palladium ou le platine, de préférence, le palladium.

3. Procédé selon l'une des revendications 1 et 2 caractérisé par le fait que le catalyseur est une zéolithe modifiée par l'élément métallique $M_1$ mis en oeuvre en une quantité telle que le pourcentage pondéral du métal par rapport à la zéolithe soit compris entre 0,1 % et 25 %, de préférence entre 3 % et 15 %.

4. Procédé selon l'une des revendications 1 à 3 caractérisé par le fait que la zéolithe est une zéolithe naturelle ou synthétique.

5. Procédé selon la revendication 4 caractérisé par le fait que la zéolithe est une zéolithe naturelle choisie parmi : la chabazite, la clinoptilolite, l'érionite, la mordénite, la phillipsite, l'offrétite.

6. Procédé selon la revendication 4 caractérisé par le fait que la zéolithe est une zéolithe synthétique choisie parmi :

   - les zéolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48.
   - les zéolithes à réseau bidimensionnel telles que la mordénite, la ferrierite,
   - les zéolithes à réseau tridimensionnel telles que la zéolithe β, la zéolithe Y, la zéolithe X, la zéolithe ZSM-5, la zéolithe ZSM-11, l'offrétite.

7. Procédé selon la revendication 6 caractérisé par le fait que la zéolithe est une zéolithe β et Y.

8. Procédé selon la revendications 1 à 7 caractérisé par le fait que le ou les éléments métalliques $M_1$ sont déposés sur la zéolithe notamment par précipitation ou par imprégnation à sec ou voie humide.

9. Procédé selon la revendication 8 caractérisé par le fait que l'on fait appel à l'oxygène pur ou l'oxygène dilué avec un gaz inerte, de préférence l'air.

10. Procédé selon l'une des revendications 8 et 9 caractérisé par le fait que la température du flux gazeux est inférieure à 350°C, et est comprise de préférence entre 100°C et 250°C.

11. Procédé selon l'une des revendications 1 à 10 caractérisé par le fait que l'on récupère un catalyseur réutilisable dans une réaction d'acylation.

12. Procédé d'acylation d'un éther aromatique qui consiste à faire réagir un éther aromatique avec un agent d'acylation, en présence d'un catalyseur, puis à récupérer le produit formé, ledit procédé étant caractérisé par le fait que l'on conduit la réaction d'acylation en présence d'une quantité efficace d'une zéolithe modifiée par un élément métallique $M_1$ choisi parmi : le ruthénium, le rhodium, le palladium, l'osmium, l'iridium, le platine selon la revendication 1.

13. Procédé selon la revendication 12 caractérisé par le fait que l'éther aromatique répond à la formule générale (I):

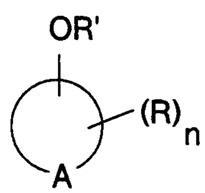

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- n est un nombre inférieur ou égal à 4.

**14.** Procédé selon l'une des revendications 12 et 13 caractérisé par le fait que l'éther aromatique répond à la formule générale (I) dans laquelle R' représente :

- un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
- un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
- un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué.
- un radical carbocyclique aromatique, de préférence monocyclique ayant généralement au moins 4 atomes de carbone, de préférence, 6 atomes de carbone dans le cycle ; ledit cycle pouvant être substitué.

**15.** Procédé selon la revendication 12 caractérisé par le fait que l'éther aromatique répond à la formule générale (I) dans laquelle R' représente un radical alkyle linéaire ou ramifié ayant de 1 à 4 atomes de carbone, de préférence un radical méthyle ou un radical phényle.

**16.** Procédé selon l'une des revendications 12 à 15 caractérisé par le fait que l'éther aromatique répond à la formule générale (I) dans laquelle le reste A représente le reste d'un composé carbocyclique aromatique, monocyclique ayant au moins 4 atomes de carbone et de préférence 6 atomes de carbone ou le reste d'un composé carbocyclique polycyclique : le reste A pouvant porter un ou plusieurs substituants sur le noyau aromatique.

**17.** Procédé selon l'une des revendications 12 à 16 caractérisé par le fait que l'éther aromatique répond à la formule (Ia):

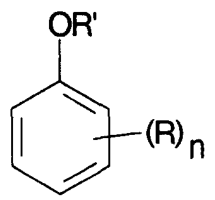

(Ia)

dans laquelle :

- n est un nombre inférieur ou égal à 4, de préférence égal à 0, 1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,
- le ou les radicaux R représentent l'un des atomes ou groupes suivants :

  . un atome d'hydrogène,
  . un radical alkyle, linéaire ou ramifié, ayant de 1 à 6 atomes de carbone, de préférence de 1 à 4 atomes

de carbone, tel que méthyle, éthyle, propyle, isopropyle, butyle, isobutyle, sec-butyle, tert-butyle,

- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un atome d'halogène, de préférence un atome de fluor, chlore ou brome, un radical trifluorométhyle.

- les radicaux R' et R et les 2 atomes successifs du cycle benzénique peuvent former entre eux, un cycle ayant de 5 à 7 atomes, comprenant éventuellement un autre hétéroatome.

**18.** Procédé selon la revendication 17 caractérisé par le fait que l'éther aromatique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence l'oxygène : les radicaux R' et R formant de préférence un radical méthylène dioxy ou éthylène dioxy.

**19.** Procédé selon l'une des revendications 17 et 18 caractérisé par le fait que l'éther aromatique répond à la formule (Ia) dans laquelle n est égal à 1, les radicaux R et R' représentant tous deux, des radicaux alkoxy, identiques ou différents.

**20.** Procédé selon la revendication 12 et 13 caractérisé par le fait que l'éther aromatique est l'anisole ou le vératrole.

**21.** Procédé selon l'une des revendications 12 à 20 caractérisé par le fait que l'agent d'acylation répond à la formule (II):

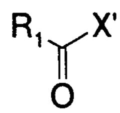

(II)

dans laquelle :

- $R_1$ représente :

  - . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 3 à 8 atomes de carbone ; un radical aliphatique saturé ou insaturé, linéaire ou ramifié, porteur d'un substituant cyclique,

- X' représente :

  - . un atome d'halogène, de préférence un atome de chlore ou de brome,
  - . un groupe hydroxyle,
  - . un radical $-O-CO-R_2$ avec $R_2$, identique ou différent de $R_1$, ayant la même signification que $R_1$ : $R_1$ et $R_2$ pouvant former ensemble un radical divalent aliphatique saturé ou insaturé, linéaire ou ramifié, ayant au moins 2 atomes de carbone.

**22.** Procédé selon la revendication 21 caractérisé par le fait que l'agent d'acylation répond à la formule (II) dans laquelle X' représente un atome de chlore et $R_1$ représente un radical alkyle linéaire ou ramifié ayant de 1 à 12 atomes de carbone : la chaîne hydrocarbonée pouvant être éventuellement interrompue par un hétéroatome ou par un groupe fonctionnel ou porteuse d'un substituant ; $R_1$ représente un radical phényle éventuellement substitué ; ou X' représente un radical $-O-CO-R_2$. dans laquelle $R_1$ et $R_2$ sont identiques, représentent un radical alkyle ayant de 1 à 4 atomes de carbone.

**23.** Procédé selon la revendication 21 et 22 caractérisé par le fait que l'agent d'acylation est choisi parmi :

- l'anhydride acétique,
- l'anhydride propanoïque,
- l'anhydride isobutyrique,

- l'anhydride trifluoroacétique,
- l'anhydride benzoïque.
- le chlorure d'acétyle,
- le chlorure de monochloroacétyle,
- le chlorure de dichloroacétyle,
- le chlorure de propanoyle,
- le chlorure d'isobutanoyle,
- le chlorure de pivaloyle,
- le chlorure de stéaroyle,
- le chlorure de crotonyle,
- le chlorure de benzoyle,
- les chlorures de chlorobenzoyle,
- le chlorure de p-nitrobenzoyle
- les chlorures de méthoxybenzoyle,
- les chlorures de naphtoyle,
- l'acide acétique.

**24.** Procédé selon l'une des revendications 12 à 23 caractérisé par le fait que le catalyseur est une zéolithe modifiée par l'élément métallique $M_1$ choisi parmi : le palladium ou le platine.

**25.** Procédé selon l'une des revendications 12 à 24 caractérisé par le fait que le catalyseur est une zéolithe modifiée par l'élément métallique $M_1$ mis en oeuvre en une quantité telle que le pourcentage pondéral du métal $M_1$ par rapport à la zéolithe soit compris entre 0,1 % et 25 %, de préférence entre 3 % et 15 %.

**26.** Procédé selon l'une des revendications 12 à 25 caractérisé par le fait que la zéolithe est une zéolithe naturelle ou synthétique.

**27.** Procédé selon la revendication 26 caractérisé par le fait que la zéolithe est une zéolithe naturelle choisie parmi : la chabazite, la clinoptilolite, l'érionite, la mordénite, la phillipsite, l'offrétite.

**28.** Procédé selon la revendication 26 caractérisé par le fait que la zéolithe est une zéolithe synthétique choisie parmi :

- les zéolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48.
- les zéolithes à réseau bidimensionnel telles que la mordénite, la ferrierite,
- les zéolithes à réseau tridimensionnel telles que la zéolithe $\beta$, la zéolithe Y, la zéolithe X, la zéolithe ZSM-5, la zéolithe ZSM-11, l'offrétite.

**29.** Procédé selon la revendication 28 caractérisé par le fait que la zéolithe est une zéolithe $\beta$ et Y.

**30.** Procédé selon l'une des revendications 12 à 29 caractérisé par le fait que l'on met en oeuvre un solvant organique choisi parmi les hydrocarbures aliphatiques et/ou aromatiques éventuellement halogénés, de préférence chlorés, les éther-oxydes aliphatiques, cycloaliphatiques ou aromatiques, les composés nitrés, les nitriles aliphatiques ou aromatiques, la tétraméthylène sulfone.

**31.** Procédé selon l'une des revendications 12 à 30 caractérisé par le fait que le rapport entre le nombre de moles d'éther aromatique et le nombre de moles d'agent d'acylation varie entre 0,1 et 10, et se situe de préférence entre 0,5 et 4.

**32.** Procédé selon l'une des revendications 12 à 31 caractérisé par le fait que dans un mode de mise en oeuvre discontinue, la quantité de catalyseur représente en poids par rapport à l'éther aromatique engagé, de 0,01 à 50 %, de préférence, de 1,0 à 20 % et dans un mode de mise en oeuvre continue, le temps de séjour du flux de matière sur le lit catalytique varie entre 5 mn et 10 heures, et de préférence, entre 15 mn et 3 heures.

**33.** Procédé selon l'une des revendications 12 à 32 caractérisé par le fait que la température à laquelle est mise en oeuvre la réaction d'acylation se situe entre 20°C et 300°C, de préférence entre 40°C et 200°C.

**34.** Procédé selon l'une des revendications 12 à 33 caractérisé par le fait que l'on effectue la régénération du catalyseur

après élimination de la phase liquide, selon le procédé décrit dans l'une des revendications 9 et 10.

**35.** Procédé selon l'une des revendications 12 à 34 caractérisé par le fait que l'on récupère un catalyseur réutilisable dans le procédé d'acylation de l'invention tel que décrit dans l'une des revendications 12 à 33.

**Patentansprüche**

**1.** Verfahren zur Regenerierung eines Katalysators auf Zeolithbasis, der in einer Acylierungsreaktion verwendet wird, dadurch gekennzeichnet, daß es darin besteht, einen heißen Gasstrom aus molekularem Sauerstoff oder einem diesen enthaltenden Gas über einen Katalysator zu leiten, der ein Zeolith ist, der durch Zugabe einer wirksamen Menge mindestens eines metallischen Elements $M_1$ modifiziert ist, das unter Ruthenium, Rhodium, Palladium, Osmium, Iridium, Platin ausgewählt ist.

**2.** Verfahren nach Anspruch 1, dadurch gekennzeichnet, daß der Katalysator ein Zeolith ist, der durch ein metallisches Element $M_1$ modifiziert ist, das unter Palladium oder Platin, vorzugsweise Palladium, ausgewählt ist.

**3.** Verfahren nach Anspruch 1 oder 2, dadurch gekennzeichnet, daß der Katalysator ein Zeolith ist, der durch ein metallisches Element $M_1$ modifiziert ist, das in einer solchen Menge eingesetzt ist, daß das Gewichtsverhältnis des Metalls zum Zeolith zwischen 0,1 % und 25 %, vorzugsweise zwischen 3 % und 15 %, liegt.

**4.** Verfahren nach einem der Ansprüche 1 bis 3, dadurch gekennzeichnet, daß der Zeolith ein natürlicher oder synthetischer Zeolith ist.

**5.** Verfahren nach Anspruch 4, dadurch gekennzeichnet, daß der Zeolith ein natürlicher Zeolith ist, der aus Chabasit, Clinoptilolith, Erionit, Mordenit, Phillipsit, Offretit ausgewählt ist.

**6.** Verfahren nach Anspruch 4, dadurch gekennzeichnet, daß der Zeolith ein synthetischer Zeolith ist, der gewählt ist aus:

- den synthetischen Zeolithen mit eindimensionalem Gitter wie Zeolith ZSM-4, Zeolith L, Zeolith ZSM-12, Zeolith ZSM-22, Zeolith ZSM-23, Zeolith ZSM-48,
- den Zeolithen mit zweidimensionalem Gitter wie Mordenit, Ferrierit,
- den Zeolithen mit dreidimensionalem Gitter wie Zeolith β, Zeolith Y, Zeolith X, Zeolith ZSM-5, Zeolith ZSM-11, Offretit.

**7.** Verfahren nach Anspruch 6, dadurch gekennzeichnet, daß der Zeolith ein Zeolith β oder Y ist.

**8.** Verfahren nach einem der Ansprüche 1 bis 7, dadurch gekennzeichnet, daß das oder die metallischen Elemente $M_1$ auf dem Zeolith insbesondere durch Ausfällung oder durch Trocken- oder Feuchtimprägnierung aufgebracht sind.

**9.** Verfahren nach Anspruch 8, dadurch gekennzeichnet, daß man reinen Sauerstoff oder mit einem inerten Gas verdünnten Sauerstoff, vorzugsweise Luft, verwendet.

**10.** Verfahren nach Anspruch 8 oder 9, dadurch gekennzeichnet, daß die Temperatur des Gasstroms unter 350 °C und vorzugsweise zwischen 100 °C und 250 °C liegt.

**11.** Verfahren nach einem der Ansprüche 1 bis 10, dadurch gekennzeichnet, daß man einen in einer Acylierungsreaktion wiederverwendbaren Katalysator gewinnt.

**12.** Verfahren zur Acylierung eines aromatischen Ethers, das darin besteht, einen aromatischen Ether mit einem Acylierungsmittel in Gegenwart eines Katalysators reagieren zu lassen, dann das erhaltene Produkt abzutrennen, wobei das genannte Verfahren dadurch gekennzeichnet ist, daß man die Acylierungsreaktion in Gegenwart einer wirksamen Menge eines Zeoliths durchführt, der durch ein metallisches Element $M_1$ modifiziert ist, das unter Ruthenium, Rhodium, Palladium, Osmium, Iridium, Platin nach Anspruch 1 ausgewählt ist.

**13.** Verfahren nach Anspruch 12, dadurch gekennzeichnet, daß der aromatische Ether der allgemeinen Formel (I)

entspricht:

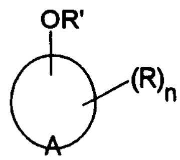

(I)

in der:

- A den Rest eines Ringes bedeutet, der einen Teil oder die Gesamtheit eines monocyclischen oder polycyclischen, carbocyclischen aromatischen Systems bildet, wobei das System mindestens eine OR'-Gruppe enthält: wobei der genannte cyclische Rest einen oder mehrere Substituenten tragen kann,
- R einen oder mehrere gleiche oder verschiedene Substituenten bedeutet,
- R' einen Kohlenwasserstoffrest mit 1 bis 24 Kohlenstoffatomen bedeutet, der ein gesättigter oder ungesättigter, linearer oder verzweigter, aliphatischer acyclischer Rest sein kann; oder ein gesättigter, ungesättigter oder aromatischer, monocyclischer oder polycyclischer, cycloaliphatischer Rest; oder ein gesättigter oder ungesättigter, linearer oder verzweigter, aliphatischer Rest, der Träger eines cyclischen Substituenten ist,
- n eine Zahl kleiner oder gleich 4 ist.

**14.** Verfahren nach Anspruch 12 oder 13, dadurch gekennzeichnet, daß der aromatische Ether der allgemeinen Formel (I) entspricht, in der R' bedeutet:

- einen gesättigten oder ungesättigten, linearen oder verzweigten, aliphatischen acyclischen Rest, vorzugsweise einen linearen oder verzweigten Alkylrest mit 1 bis 12 Kohlenstoffatomen, vorzugsweise mit 1 bis 6 Kohlenstoffatomen: wobei die Kohlenwasserstoffkette gegebenenfalls durch ein Heteroatom, eine funktionelle und/oder eine einen Substituenten tragende Gruppe unterbrochen sein kann,
- einen gesättigten oder ungesättigten linearen oder verzweigten, aliphatischen acyclischen Rest, der Träger eines cyclischen und eventuell substituierten Substituenten ist: wobei der genannte acyclische Rest mit dem Ring durch ein valentes Bindeglied, ein Heteroatom oder eine funktionelle Gruppe verbunden sein kann,
- einen gesättigten oder 1 oder 2 ungesättigte Stellen im Ring enthaltenden carbocyclischen Rest mit im allgemeinen 3 bis 8 Kohlenstoffatomen, vorzugsweise 6 Kohlenstoffatomen, im Ring; wobei der Ring substituiert sein kann,
- einen aromatischen, vorzugsweise monocyclischen, carbocyclischen Rest mit im allgemeinen mindestens 4 Kohlenstoffatomen, vorzugsweise 6 Kohlenstoffatomen, im Ring; wobei der Ring substituiert sein kann.

**15.** Verfahren nach Anspruch 12, dadurch gekennzeichnet, daß der aromatische Ether der allgemeinen Formel (I) entspricht, in der R' einen linearen oder verzweigten Alkylrest mit 1 bis 4 Kohlenstoffatomen, vorzugsweise einen Methyl- oder Phenylrest, bedeutet.

**16.** Verfahren nach einem der Ansprüche 12 bis 15, dadurch gekennzeichnet, daß der aromatische Ether der allgemeinen Formel (I) entspricht, in der der Rest A den Rest einer aromatischen, monocyclischen, carbocyclischen Verbindung mit mindestens 4 Kohlenstoffatomen und vorzugsweise 6 Kohlenstoffatomen oder den Rest einer polycyclischen, carbocyclischen Verbindung bedeutet: wobei der Rest A einen oder mehrere Substituenten auf dem aromatischen Ring tragen kann.

**17.** Verfahren nach einem der Ansprüche 12 bis 16, dadurch gekennzeichnet, daß der aromatische Ether der Formel (Ia) entspricht:

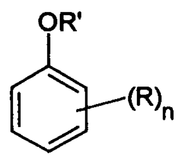

(Ia)

in der:

- n eine Zahl kleiner oder gleich 4, vorzugsweise 0, 1 oder 2, ist,
- der Rest R' einen linearen oder verzweigten Alkylrest mit 1 bis 6 Kohlenstoffatomen, vorzugsweise mit 1 bis 4 Kohlenstoffatomen, bedeutet wie Methyl, Ethyl, Propyl, Isopropyl, Butyl, Isobutyl, sec.-Butyl, tert.-Butyl oder Phenyl,
- der oder die Reste R eine der folgenden Atome oder Gruppen bedeuten:
    - ein Wasserstoffatom,
    - einen linearen oder verzweigten Alkylrest mit 1 bis 6 Kohlenstoffatomen, vorzugsweise mit 1 bis 4 Kohlenstoffatomen, wie Methyl, Ethyl, Propyl, Isopropyl, Butyl, Isobutyl, sec.-Butyl, tert.-Butyl,
    - einen linearen oder verzweigten Alkoxyrest mit 1 bis 6 Kohlenstoffatomen, vorzugsweise mit 1 bis 4 Kohlenstoffatomen, wie die Methoxy-, Ethoxy-, Propoxy-, Isopropoxy-, Butoxy-, Isobutoxy-, sec.-Butoxy-, tert.-Butoxyreste,
    - ein Halogenatom, vorzugsweise ein Fluor-, Chlor- oder Bromatom, ein Trifluormethylrest,
- die Reste R' und R und die 2 aufeinanderfolgenden Atome des Benzolrings untereinander einen Ring mit 5 bis 7 Atomen bilden können, der gegebenenfalls ein anderes Heteroatom aufweist.

**18.** Verfahren nach Anspruch 17, dadurch gekennzeichnet, daß der aromatische Ether der Formel (Ia) entspricht, in der n größer oder gleich 1 ist, die Reste R' und R und die 2 aufeinanderfolgenden Atome des Benzolrings untereinander durch einen Alkylen-, Alkenylen- oder Alkenylidenrest mit 2 bis 4 Kohlenstoffatomen verbunden sein können, um einen gesättigten, ungesättigten oder aromatischen Heteroring mit 5 bis 7 Kohlenstoffatomen zu bilden, in dem ein oder mehrere Kohlenstoffatome durch ein Heteroatom, vorzugsweise Sauerstoff, ersetzt sein können: wobei die Reste R' und R vorzugsweise einen Methylendioxy- oder Ethylendioxyrest bilden.

**19.** Verfahren nach Anspruch 17 oder 18, dadurch gekennzeichnet, daß der aromatische Ether der Formel (Ia) entspricht, in der n gleich 1 ist und die Reste R und R' beide gleiche oder verschiedene Alkoxyreste bedeuten.

**20.** Verfahren nach Anspruch 12 oder 13, dadurch gekennzeichnet, daß der aromatische Ether Anisol oder 1,2-Dimethoxybenzol ist.

**21.** Verfahren nach einem der Ansprüche 12 bis 20, dadurch gekennzeichnet, daß das Acylierungsmittel der Formel (II) entspricht:

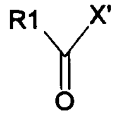

(II)

in der:

- $R_1$ bedeutet:
    - einen gesättigten oder ungesättigten, linearen oder verzweigten, aliphatischen Rest mit 1 bis 24 Kohlenstoffatomen; einen gesättigten, ungesättigten oder aromatischen, monocyclischen oder polycyclischen, cycloaliphatischen Rest mit 3 bis 8 Kohlenstoffatomen; einen gesättigten oder ungesättigten, linearen oder verzweigten, aliphatischen Rest, der Träger eines cyclischen Substituenten ist,
- X' bedeutet:
    - ein Halogenatom, vorzugsweise ein Chlor- oder Bromatom,
    - eine Hydroxylgruppe,
    - einen $-O-CO-R_2$-Rest mit $R_2$, das gleich mit oder unterschiedlich zu $R_1$ ist, mit derselben Bedeutung wie $R_1$: wobei $R_1$ und $R_2$ zusammen einen gesättigten oder ungesättigten, linearen oder verzweigten, zweiwertigen aliphatischen Rest mit mindestens 2 Kohlenstoffatomen bilden können.

**22.** Verfahren nach Anspruch 21, dadurch gekennzeichnet, daß das Acylierungsmittel der Formel (II) entspricht, in der X' ein Chloratom und $R_1$ einen linearen oder verzweigten Alkylrest mit 1 bis 12 Kohlenstoffatomen bedeutet: wobei die Kohlenwasserstoffkette gegebenenfalls durch ein Heteroatom oder durch eine funktionelle oder einen Substituenten tragende Gruppe unterbrochen sein kann; $R_1$ einen gegebenenfalls substituierten Phenylrest bedeutet; oder X' einen -O-CO-$R_2$-Rest bedeutet, wobei die gleichen $R_1$ und $R_2$ einen Alkylrest mit 1 bis 4 Kohlenstoffatomen bedeuten.

**23.** Verfahren nach Anspruch 21 oder 22, dadurch gekennzeichnet, daß das Acylierungsmittel gewählt ist aus:

- Essigsäureanhydrid,
- Propionsäureanhydrid,
- Isobuttersäureanhydrid,
- Trifluoressigsäureanhydrid,
- Benzoesäureanhydrid,
- Acetylchlorid,
- Monochloracetylchlorid,
- Dichloracetylchlorid,
- Propanoylchlorid,
- Isobuttersäurechlorid,
- Pivaloylchlorid,
- Stearinsäurechlorid,
- Crotonoylchlorid,
- Benzoylchlorid,
- den Chlorbenzoylchloriden,
- p-Nitrobenzoylchlorid,
- den Methoxybenzoylchloriden,
- den Naphtoylchloriden,
- Essigsäure.

**24.** Verfahren nach einem der Ansprüche 12 bis 23, dadurch gekennzeichnet, daß der Katalysator ein Zeolith ist, das durch das metallische Element $M_1$ modifiziert ist, das gewählt ist aus: Palladium oder Platin.

**25.** Verfahren nach einem der Ansprüche 12 bis 24, dadurch gekennzeichnet, daß der Katalysator ein Zeolith ist, der durch das metallische Element $M_1$ modifiziert ist, das in einer solchen Menge eingesetzt ist, daß der Gewichtsanteil des Metalls $M_1$ im Verhältnis zum Zeolithen zwischen 0,1 % und 25 %, vorzugsweise zwischen 3 % und 15 %, liegt.

**26.** Verfahren nach einem der Ansprüche 12 bis 25, dadurch gekennzeichnet, daß der Zeolith ein natürlicher oder synthetischer Zeolith ist.

**27.** Verfahren nach Anspruch 26, dadurch gekennzeichnet, daß der Zeolith ein natürlicher Zeolith ist, der unter Chabasit, Clinoptilolith, Erionit, Mordenit, Phillipsit, Offretit ausgewählt ist.

**28.** Verfahren nach Anspruch 26, dadurch gekennzeichnet, daß der Zeolith ein synthetischer Zeolith ist, der gewählt ist aus:

- den synthetischen Zeolithen mit eindimensionalem Gitter wie Zeolith ZSM-4, Zeolith L, Zeolith ZSM-12, Zeolith ZSM-22, Zeolith ZSM-23, Zeolith ZSM-48,
- den Zeolithen mit zweidimensionalem Gitter wie Mordenit, Ferrierit,
- den Zeolithen mit dreidimensionalem Gitter wie Zeolith β, Zeolith Y, Zeolith X, Zeolith ZSM-5, Zeolith ZSM-11, Offretit.

**29.** Verfahren nach Anspruch 28, dadurch gekennzeichnet, daß der Zeolith ein Zeolith β oder Y ist.

**30.** Verfahren nach einem der Ansprüche 12 bis 29, dadurch gekennzeichnet, daß man ein organisches Lösungsmittel einsetzt, das aus den aliphatischen und/oder aromatischen und gegebenenfalls halogenisierten, vorzugsweise chlorierten, Kohlenwasserstoffen, den aliphatischen, cycloaliphatischen oder aromatischen Etheroxiden, den nitrierten Verbindungen, den aliphatischen oder aromatischen Nitrilen oder Tetramethylensulfon gewählt ist.

31. Verfahren nach einem der Ansprüche 12 bis 30, dadurch gekennzeichnet, daß das Verhältnis zwischen der Molzahl des aromatischen Ethers und der Molzahl des Acylierungsmittels zwischen 0,1 und 10 und vorzugsweise zwischen 0,5 und 4 schwankt.

32. Verfahren nach einem der Ansprüche 12 bis 31, dadurch gekennzeichnet, daß bei einer unterbrochenen Art der Durchführung die Menge des Katalysators bezogen auf das Gewicht im Verhältnis zum verwendeten aromatischen Ether 0,01 bis 50 %, vorzugsweise 1,0 bis 20 %, beträgt und bei einer kontinuierlichen Art der Durchführung die Verweilzeit des Materiestroms über dem katalytischen Bett zwischen 5 Minuten und 10 Stunden, vorzugsweise zwischen 15 Minuten und 3 Stunden, schwankt.

33. Verfahren nach einem der Ansprüche 12 bis 32, dadurch gekennzeichnet, daß die Temperatur, bei der die Acylierungsreaktion stattfindet, zwischen 20 °C und 300 °C, vorzugsweise zwischen 40 °C und 200 °C, liegt.

34. Verfahren nach einem der Ansprüche 12 bis 33, dadurch gekennzeichnet, daß man die Regeneration des Katalysators nach Entfernen der flüssigen Phase gemäß dem in den Ansprüchen 9 und 10 beschriebenen Verfahren durchführt.

35. Verfahren nach einem der Ansprüche 12 bis 34, dadurch gekennzeichnet, daß man einen im erfindungsgemäßen und in den Ansprüchen 12 bis 33 beschriebenen Acylierungsverfahren wiederverwendbaren Katalysator gewinnt.

**Claims**

1. Process for regeneration of a catalyst based on a zeolite, employed in an acylation reaction, characterized in that it consists in sending a hot gaseous stream of molecular oxygen or of a gas containing it over a catalyst which is a zeolite modified by addition of an effective quantity of at least one metallic element $M_1$ chosen from ruthenium, rhodium, palladium, osmium, iridium and platinum.

2. Process according to Claim 1, characterized in that the catalyst is a zeolite modified with the metallic element $M_1$ chosen from palladium or platinum, preferably palladium.

3. Process according to either of Claims 1 and 2, characterized in that the catalyst is a zeolite modified with the metallic element $M_1$ used in a quantity such that the percentage by weight of the metal relative to the zeolite is between 0.1 % and 25 %, preferably between 3 % and 15 %.

4. Process according to one of Claims 1 to 3, characterized in that the zeolite is a natural or synthetic zeolite.

5. Process according to Claim 4, characterized in that the zeolite is a natural zeolite chosen from chabazite, clinoptilolite, erionite, mordenite, phillipsite and offretite.

6. Process according to Claim 4, characterized in that the zeolite is a synthetic zeolite chosen from:

- synthetic zeolites with a one-dimensional network such as zeolite ZSM-4, zeolite L, zeolite ZSM-12, zeolite ZSM-22, zeolite ZSM-23 and zeolite ZSM-48,
- zeolites with a two-dimensional network, such as mordenite and ferrierite,
- zeolites with a three-dimensional network such as zeolite β, zeolite Y, zeolite X, zeolite ZSM-5, zeolite ZSM-11 and offretite.

7. Process according to Claim 6, characterized in that the zeolite is a zeolite β and Y.

8. Process according to Claims 1 to 7, characterized in that the metallic element(s) $M_1$ are deposited on the zeolite especially by precipitation or by impregnation by a dry or wet route.

9. Process according to Claim 8, characterized in that pure oxygen or oxygen diluted with an inert gas, preferably air, is used.

10. Process according to either of Claims 8 and 9, characterized in that the temperature of the gaseous stream is lower than 350°C and is preferably between 100°C and 250°C.

**11.** Process according to one of Claims 1 to 10, characterized in that a catalyst which can be reemployed in an acylation reaction is recovered.

**12.** Process for acylation of an aromatic ether, which consists in reacting an aromatic ether with an acylating agent, in the presence of a catalyst, and in then recovering the product formed, the said process being characterized in that the acylation reaction is conducted in the presence of an effective quantity of a zeolite modified with a metallic element $M_1$ chosen from ruthenium, rhodium, palladium, osmium, iridium and platinum according to Claim 1.

**13.** Process according to Claim 12, characterized in that the aromatic ether corresponds to the general formula (I):

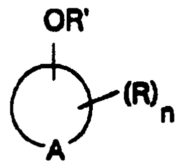

(I)

in which:

- A symbolizes the residue of a ring forming all or part of a monocyclic or polycyclic aromatic carbocyclic system, the system including at least one OR' group, it being possible for the said cyclic residue to carry one or more substituents,
- R denotes one or a number of identical or different substituents,
- R' denotes a hydrocarbon radical containing from 1 to 24 carbon atoms, which may be a linear or branched, saturated or unsaturated, acyclic aliphatic radical, a saturated or unsaturated cycloaliphatic or monocyclic or polycyclic aromatic radical, or a linear or branched, saturated or unsaturated, aliphatic radical carrying a cyclic substituent,
- n is a number smaller than or equal to 4.

**14.** Process according to either of Claims 12 and 13, characterized in that the aromatic ether corresponds to the general formula (I) in which R' denotes:

- a linear or branched, saturated or unsaturated, acyclic aliphatic radical, preferably a linear or branched alkyl radical containing from 1 to 12 carbon atoms, preferably from 1 to 6 carbon atoms, it being possible for the hydrocarbon chain to be optionally interrupted by a heteroatom, a functional group and/or to carry a substituent,
- a linear or branched, saturated or unsaturated, acyclic aliphatic radical carrying an optionally substituted cyclic substituent, it being possible for the said acyclic radical to be linked to the ring by a valency bond, a heteroatom or a functional group,
- a carbocyclic radical which is saturated or includes 1 or 2 unsaturations in the ring, generally containing from 3 to 8 carbon atoms, preferably 6 carbon atoms in the ring, it being possible for the said ring to be substituted,
- a preferably monocyclic aromatic carbocyclic radical generally containing at least 4 carbon atoms, preferably 6 carbon atoms, in the ring, it being possible for the said ring to be substituted.

**15.** Process according to Claim 12, characterized in that the aromatic ether corresponds to the general formula (I) in which R' denotes a linear or branched alkyl radical containing from 1 to 4 carbon atoms, preferably a methyl radical or a phenyl radical.

**16.** Process according to one of Claims 12 to 15, characterized in that the aromatic ether corresponds to the general formula (I) in which the residue A denotes the residue of a monocyclic aromatic carbocyclic compound containing at least 4 carbon atoms and preferably 6 carbon atoms or the residue of a polycyclic carbocyclic compound, it being possible for the residue A to carry one or more substituents on the aromatic nucleus.

**17.** Process according to one of Claims 12 to 16, characterized in that the aromatic ether corresponds to the formula (Ia):

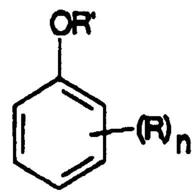

(Ia)

in which:

- n is a number smaller than or equal to 4, preferably equal to 0, 1 or 2,
- the radical R' denotes a linear or branched alkyl radical containing from 1 to 6 carbon atoms, preferably from 1 to 4 carbon atoms, such as methyl, ethyl, propyl, isopropyl, butyl, isobutyl, sec-butyl, tert-butyl or a phenyl radical,
- the radical(s) R denote one of the following atoms or groups:
    - a hydrogen atom,
    - a linear or branched alkyl radical containing from 1 to 6 carbon atoms, preferably from 1 to 4 carbon atoms, such as methyl, ethyl, propyl, isopropyl, butyl, isobutyl, sec-butyl or tert-butyl,
    - a linear or branched alkoxy radical containing from 1 to 6 carbon atoms, preferably from 1 to 4 carbon atoms, such as methoxy, ethoxy, propoxy, isopropoxy, butoxy, isobutoxy, sec-butoxy or tert-butoxy radicals,
    - a halogen atom, preferably a fluorine, chlorine or bromine atom, or a trifluoromethyl radical,
- the radicals R' and R and the 2 successive atoms of the benzene ring can together form a ring containing from 5 to 7 atoms, optionally including another heteroatom.

**18.** Process according to Claim 17, characterized in that the aromatic ether corresponds to the formula (Ia) in which n is greater than or equal to 1, the radicals R' and R and the 2 successive atoms of the benzene ring may be linked together by an alkylene, alkenylene or alkenylidene radical containing from 2 to 4 carbon atoms to form a saturated, unsaturated or aromatic heterocyclic ring containing from 5 to 7 carbon atoms, in which one or more carbon atoms may be replaced by a heteroatom, preferably oxygen, the radicals R' and R preferably forming a methylenedioxy or ethylenedioxy radical.

**19.** Process according to either of Claims 17 and 18, characterized in that the aromatic ether corresponds to the formula (Ia) in which n is equal to 1, the radicals R and R' both denoting identical or different alkoxy radicals.

**20.** Process according to Claim 12 and 13, characterized in that the aromatic ether is anisole or veratrole.

**21.** Process according to one of Claims 12 to 20, characterized in that the acylating agent corresponds to the formula (II):

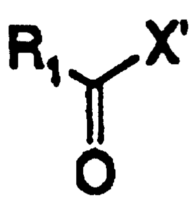

(II)

in which:

- $R_1$ denotes:
    - a linear or branched, saturated or unsaturated, aliphatic radical containing from 1 to 24 carbon atoms, a saturated, unsaturated cycloaliphatic or a monocyclic or polycyclic aromatic radical containing from 3 to 8 carbon atoms, or a linear or branched, saturated or unsaturated, aliphatic radical carrying a cyclic substituent,

- X' denotes:

  - a halogen atom, preferably a chlorine or bromine atom,
  - a hydroxyl group,
  - a radical -O-CO-$R_2$ with $R_2$, identical or different from $R_1$ having the same meaning as $R_1$, it being possible for $R_1$ and $R_2$ to form together a linear or branched, saturated or unsaturated aliphatic divalent radical containing at least 2 carbon atoms.

**22.** Process according to Claim 21, characterized in that the acylating agent corresponds to the formula (II) in which X' denotes a chlorine atom and $R_1$ denotes a linear or branched alkyl radical containing from 1 to 12 carbon atoms, it being possible for the hydrocarbon chain to be optionally interrupted by a heteroatom or by a functional group or to carry a substituent, $R_1$ denotes an optionally substituted phenyl radical, or X' denotes a radical -O-CO-$R_2$ in which $R_1$ and $R_2$ are identical and denote an alkyl radical containing from 1 to 4 carbon atoms.

**23.** Process according to Claim 21 and 22, characterized in that the acylating agent is chosen from:

- acetic anhydride,
- propanoic anhydride,
- isobutyric anhydride,
- trifluoroacetic anhydride,
- benzoic anhydride,
- acetyl chloride,
- monochloroacetyl chloride,
- dichloroacetyl chloride,
- propanoyl chloride,
- isobutanoyl chloride,
- pivaloyl chloride,
- stearoyl chloride,
- crotonyl chloride,
- benzoyl chloride,
- chlorobenzoyl chlorides,
- p-nitrobenzoyl chloride,
- methoxybenzoyl chlorides,
- naphthoyl chlorides,
- acetic acid.

**24.** Process according to one of Claims 12 to 23, characterized in that the catalyst is a zeolite modified with the metallic element $M_1$ chosen from palladium or platinum.

**25.** Process according to one of Claims 12 to 24, characterized in that the catalyst is a zeolite modified with the metallic element $M_1$ used in a quantity such that the percentage by weight of the metal $M_1$ relative to the zeolite is between 0.1 % and 25 %, preferably between 3 % and 15 %.

**26.** Process according to one of Claims 12 to 25, characterized in that the zeolite is a natural or synthetic zeolite.

**27.** Process according to Claim 26, characterized in that the zeolite is a natural zeolite chosen from chabazite, clinoptilolite, erionite, mordenite, phillipsite and offretite.

**28.** Process according to Claim 26, characterized in that the zeolite is a synthetic zeolite chosen from:

- synthetic zeolites with a one-dimensional network such as zeolite ZSM-4, zeolite L, zeolite ZSM-12, zeolite ZSM-22, zeolite ZSM-23 and zeolite ZSM-48,
- zeolites with a two-dimensional network, such as, mordenite and ferrierite,
- zeolites with a three-dimensional network such as zeolite β, zeolite Y, zeolite X, zeolite ZSM-5, zeolite ZSM-11 and offretite.

**29.** Process according to Claim 28, characterized in that the zeolite is a zeolite β and Y.

**30.** Process according to one of Claims 12 to 29, characterized in that an organic solvent is used, chosen from optionally halogenated, preferably chlorinated aliphatic and/or aromatic hydrocarbons, aliphatic, cycloaliphatic or aromatic ethers, nitro compounds, aliphatic or aromatic nitriles and tetramethylene sulphone.

**31.** Process according to one of Claims 12 to 30, characterized in that the ratio of the number of moles of aromatic ether to the number of moles of acylating agent varies between 0.1 and 10 and preferably lies between 0.5 and 4.

**32.** Process according to one of Claims 12 to 31, characterized in that in a noncontinuous method of operation the quantity of catalyst represents from 0.01 to 50 %, preferably from 1.0 to 20 % by weight relative to the aromatic ether introduced and, in a continuous method of operation, the residence time of the flow of material on the catalyst bed varies between 5 min and 10 hours and preferably between 15 min and 3 hours.

**33.** Process according to one of Claims 12 to 32, characterized in that the temperature at which the acylation reaction is carried out lies between 20°C and 300°C, preferably between 40°C and 200°C.

**34.** Process according to one of Claims 12 to 33, characterized in that the regeneration of the catalyst is performed after removal of the liquid phase, according to the process described in either of Claims 9 and 10.

**35.** Process according to one of Claims 12 to 34, characterized in that a catalyst which can be reemployed in the acylation process of the invention as described in one of Claims 12 to 33 is recovered.